# EUROPEAN PATENT SPECIFICATION

(11) **EP 1 225 913 B1**
(45) Date of publication and mention of the grant of the patent: **17.08.2005**
(21) Application number: 00980159.8
(22) Date of filing: 02.11.2000
(51) Int. Cl.: A61K 38/44, A61K 33/00, A61M 15/00

(54) **USE OF NITRIC OXIDE FOR THE TREATMENT OF AIRWAY CONSTRICTION**
VERWENDUNG VON STICKSTOFFOXID ZUR BEHANDLUNG VON ATEMWEGS-VERENGUNGEN
UTILISATION DE MONOXYDE D'AZOTE POUR LE TRAITEMENT D'UNE CONSTRICTION DU CONDUIT AERIEN

(30) Priority: 03.11.1999 SE 9903985
(43) Date of publication of application: 31.07.2002
(73) Proprietor: AGA AB (publ), 181 81 Lindingö (SE)
(72) Inventor: HJOBERG, Josephine, 460 64 Fränderfors (SE); HEDENSTIERNA, Göran, S-182 64 Djursholm (SE); HÖGMAN, Marieann, S-747 93 Alunda (SE)
(74) Representative: Larsson, Kjell
(86) International application number: PCT/SE2000/002153
(87) International publication number: WO 2001/032202

(56) References cited:
- WO-A1-95/10185
- WO-A1-96/39409
- WO-A1-99/37616
- US-A- 5 485 827
- US-A- 5 873 359
- DATABASE MEDLINE [Online] SEKI S. ET AL.: 'Superoxide anion scavengers restore NO-mediated pulmonary vasodilation after lungtransplantation', XP002954906 Retrieved from STN Database accession no. 1999104010 & AMERICAN JOURNAL OF PHYSIOLOGY vol. 276, no. 1 PT 2, January 1999, pages H42 - H46

## Description

### Field of the invention

The present invention is within the field of medicaments for the treatment of airway constriction in a mammal, especially man. Particularly, the invention is convenient for enhancing the effect of nitric oxide treatment or producing an effect of nitric oxide in non-responders.

### Background of the invention

Nitric oxide relaxes airway smooth muscle (Belvisi MG, Stretton CD, Barnes PJ. Nitric oxide is the endogeneous neurotransmitter of bronchodilator nerves in human airways. Eur. J. Pharmacol. 1992; 210: 221-222), and inhalation of exogenous nitric oxide attenuates bronchoconstriction in the response to various agents in laboratory animals and humans (Dupuy PM, Shore SA, Drazen JM, Frostell C, Hill WA, Zapol WM. Bronchodilator action of inhaled nitric oxide in guinea pigs. J.Clin.Invest. 1992; 90:421-428; Högman M, Frostell C, Arnberg H, Hedenstierna G. Inhalation of nitric oxide modulates methacholine-induced bronchoconstriction in the rabbit. Eur.Respir.J. 1993; 6:177-180; Högman M, Frostell CG, Hedenström H, Hedenstierna G. Inhalation of nitric oxide modulates adult human bronchial tone. Am.Rev.Respir.Dis. 1993; 148:1474-1478). Attempts have been made to relax constricted airways of asthmatics by nitric oxide inhalation. EP 560 928, US 5,485,827 and 5,873,359 disclose use of nitric oxide for treating bronchoconstriction and pulmonary vasoconstriction. It has however been found that the effect of the treatment showed great inter- and intra- individual variability, and some asthmatics were non responders with no improvement at all by nitric oxide (Högman M, Frostell CG, Hedenström H, Hedenstierna G. Am.Rev.Respir.Dis. 1993; 148:1474-1478).

Patients with asthma have varying degrees of airway wall oedema (Jeffery. P.K. (1998). Airway pathology in asthma. In *Asthma. Basic mechanisms and clinical management.* eds. Barnes, P.J., Rodger, I.W. and Thompson, N.C. pp. 47-64 San Diego, CA, U.S.A.: Academic Press.) It was suggested that the oedema could be the explanation for the variations in the effect of nitric oxide seen in astmatics. In a study in rabbits the effect of nitric oxide on methacholine-induced bronchoconstriction is abolished by increasing the osmolarity of the surface liquid by nebulising hypertonic saline (Högman M, Hjoberg J, Hedenstierna G. Increased airway osmolarity inhibits the action of nitric oxide in the rabbit. Eur.Respir.J. 1998; 12:1313-1317). Also, in guinea pig trachea *in vitro* the effect of nitric oxide donors is attenuated by adding NaCl to elevate the osmolarity of the buffer perfusing the lumen of the trachea (Hjoberg J, Högman M, Hedenstierna G. Hyperosmolarity reduces the relaxing potency of nitric oxide donors in guinea-pig trachea. Br.J.Pharmacol. 1999; 127:391-396).

Therefore the main problem behind this invention was to establish why the dilatory effect of nitric oxide on airways is generally reduced when the airway surface is exposed to hyperosmolarity. Based on the findings thereof a solution to said problem has now been found, viz. a group of compounds having the ability of counteracting said reduced effect of nitric oxide.

Accordingly one object of the present invention is to provide suitable compounds to be used for relaxing the airways.

More specifically, the purpose of said compounds is to accomplish a relaxation of the airways.

Another object of the invention is to provide suitable compounds to be used to counteract reduced relaxing effects of nitric oxide when used alone, preferably for use in non-responders to nitric oxide alone.

A further object of the invention is to accomplish the use of a pure inhalable medicament.

Other objects of the invention should be apparent to a person skilled in the art after having read the description below.

### Summary of the invention

The above-mentioned objects as well as other objects of the invention, which can be gathered by a person skilled in the art after having studied the description below, are accomplished by the use, method and pharmaceutical preparation defined in the accompanying claims.

More specifically, according to a first aspect of the invention there is provided a use of inhalable nitric oxide(NO) in the form of gaseous nitric oxide or of a nitric oxide donor, in combination with a superoxide anion scavenger for the manufacture of a medicament for treating airway constriction in a mammal, especially man, said combination being used in a therapeutically effective amount to accomplish relaxation of said airway constriction.

Thus, according to the present invention it was surprisingly found that the reduced relaxing effect of exogenous nitric oxide, found in trachea subjected to intraluminal hyperosmolarity, is, at least partly, due to superoxide anions.

Superoxide anions can be produced by several cells in isolated guinea pig trachea (Sadeghi-Hashjin G, Henricks PA, Folkerts G, Muis T, Garssen J, Nijkamp FP. Role of the epithelial layer in the generation of superoxide anion by the guinea-pig isolated trachea. Mediators.Inflamm. 1998; 7:35-40.) and they will rapidly react with nitric oxide to form peroxynitrite (Huie RE, Padmaja S. The reaction of NO with superoxide. Free Radic.Res.Commun. 1993; 18:195-199; Saran M, Michel C, Bors W. Reaction of NO with O2- . implications for the action of endothelium- derived relaxing factor (EDRF). Free Radic.Res.Commun. 1990; 10:221-226.). Peroxynitrite is known to cause tissue injury (Kooy NW, Royall JA, Ye YZ, Kelly DR, Beckman JS. Evidence for in vivo peroxynitrite production in human acute lung injury. Am.J.Respir.Crit.Care Med. 1995; 151:1250-1254; Beckman JS, Beckman TW, Chen J, Marshall PA, Freeman BA. Apparent hydroxyl radical production by peroxynitrite: implications for endothelial injury from nitric oxide and superoxide. Proc.Natl.Acad.Sci.U.S.A. 1990; 87:1620-1624.) and to induce airway hyper-responsiveness (Sadeghi-Hashjin G, Folkerts G, Henricks PA, Verheyen AK, van der Linde HJ, van Ark I, Coene A, Nijkamp FP. Peroxynitrite induces airway hyperresponsiveness in guinea pigs in vitro and in vivo. Am.J.Respir.Crit.Care Med. 1996; 153:1697-1701.).

More specifically, it was found that when a scavenger of superoxide anions, in the form of a superoxide anion scavenger, was used in combination with a nitric oxide donor, the relaxing effect of the nitric oxide was potentiated, or the reduced effect of nitric oxide alone was reversed. In other words, the problem with poor response or non-response to nitric oxide treatment of airway constriction appears to be an inactivation of the nitric oxide molecule.

As to prior art in this respect, for instance the following can be referred to.

WO 96/39409 discloses compounds that oxidize and/or reduce superoxides. The compounds can be administered with nitric oxide or nitric oxide adducts. The compounds are useful for treating inflammatory disorders in mammals, particularly humans. Asthma is mentioned as one of many listed inflammatory disorders. However, there is no information of improved dilatory effect on the airways, i.e. asthma bronchiale is not referred to.

The use of superoxide ion scavengers for a medical purpose is also disclosed in WO 99/37616. However, the treatment disclosed relates to conditions associated with oxidative stress or endothelial dysfunction. Furthermore, the effect referred to is intracellular. In other words, there are differences in kind between the two uses, respectively. Thus, the reference to asthma in a listing of conditions in WO 99/37616 relates to a condition associated with oxidative stress or endothelial dysfunction, which is the case in airway inflammation and vascular dysfunction disorders, respectively, and not to airway constriction as in the present invention. The present invention is not dependent on any endothelial dysfunction. Rather the effect seems to be an epithelial one. Furthermore, it is generally extracellular.

Endothelium-dependent activity of a superoxide anion scavenger is also referred to in Medline, accession No. 1999104010. Furthermore, said activity is disclosed in connection with lung transplantation only and is limited to a pulmonary vascular effect.

US 5,747,026 relates to a method of delivering antioxidants to cells and tissues. The compounds are delivered in pH-sensitive liposomes. This method of delivering antioxidants to tissues is said to, among other things, prevent vasospasm and pulmonary toxicity of diverse oxidants and preserve the action of nitric oxide. The experimental data provided only show that antioxidants per se exert a vascular relaxation effect in certain animal experiments. There are no data presented that antioxidants might strengthen the relaxing effect on airways of inhaled nitric oxide.

In Pediatr. Res. 1999; 45: 293 A there is disclosed a study of the effect of rhSOD and inhaled gaseous NO on pulmonary hypertension in a lamb model. However, there is no suggestion whatsoever concerning any effects on the airways.

### Detailed description of the invention

The combination of nitric oxide and a superoxide anion scavenger according to the present invention can be used for the manufacture of a medicament for treating all types of constriction challenges in the airways. Such constriction challenges can be the result of airway hyperreactivity, asthma and other conditions causing airway constriction.

A preferred embodiment of the present invention is in the manufacture of a medicament for treating airway constriction associated with asthma bronchiale, especially an acute condition of asthma bronchiale.

An especially preferable embodiment of the invention is represented by the treatment of a mammal who has been shown to be non-responding to inhalation of nitric oxide (gaseous or in the form of a donor) only. This embodiment is of significant clinical importance since within different patient groups treated with inhaled nitric oxide there is generally a large group of non-responders.

According to one embodiment of the invention nitric oxide is used in gaseous, inhalable form. Inhalation of gaseous nitric oxide may represent a great advantage in therapy, e.g. in comparison with a non-gaseous nitric oxide-donor, as the gas has no particles or droplets to disperse and transport to the respiratory tract. Gases have long free-diffusion pathways, easily bypass obstructions (such as constricted airways), and dissolve directly in tissue without causing impaction bronchospasm. The beneficial effect of NO gas on bronchial smooth muscle tone is observed immediately following inhalation, making NO a useful first defense against bronchospasm that can be followed, if desired, by inhalation of longer-acting agents.

According to another embodiment of the invention, however, as defined above, the nitric oxide is administered in the form of a nitric oxide donor, i.e. a compound that act by releasing nitric oxide. Known nitric oxide releasing compounds useful in practice of the invention are nitroso or nitrosyl compounds such as S-nitroso-N-acetylpenicillamine, S-nitroso-L-cysteine and nitrosoguanidine,characterized by an -NO moitey that is spontaneously released or otherwise transferred from the compound under physiological conditions such as obtained in the lung. Other compounds are compounds in which NO is a ligand on a transition metal complex and as such is readily released or transferred from the compound under physiological conditions, e.g. nitroprusside, NO-ferredoxin, or an NO-heme complex. Further suitable nitrogen-containing compounds are compounds which are metabolized by enzymes endogenous to the respiratory and/or vascular system to produce the NO radical, e.g. arginine, glycerol trinitrate, isoamylnitrite, inorganic nitrite, azide and hydroxylamine. Such types of nitric oxide releasing compounds and method for their synthesis are well known in the art. Preferably the nitric oxide donor is a compound that releases nitric oxide in such a way that only the airways and the pulmonary vessels are affected.

The nitric oxide donor used in the invention may be administered as a powder(i.e. finely divided solid, either provided pure or as a mixture with a biologically compatible carrier powder, or with one or more additional therapeutic compounds) or as a liquid(i.e. dissolved or suspended in a biologically compatible liquid carrier, optionally mixed with one or more additional therapeutic compounds), and can conveniently be inhaled in nebulized form (preferably including particles or droplets having a diameter of less than 10 µm). Carrier liquids and powders that are suitable for inhalation are commonly used in traditional asthma inhalation therapeutics and thus well known in the art. The optimal dosage range can be determined by routine procedures known to the skilled man.

The superoxide anion scavenger to be used in the invention can be any compound recognized as being suitable to mammalian, especially human, use, which can be conveniently administered. Said superoxide anion scavenger can be an enzyme having the ability of removing superoxide anions. Superoxide dismutase is a well known, widely distributed enzyme that is a preferred compound for the use according to the invention. The superoxide anion scavenger can, however, also be another compound having the ability of scavenging superoxide anions. Example of such compounds are antioxidants, e.g. vitamin E, vitamin C, bilirubin, urate, butylated hydroxytoluene, butylated hydroxyanisole, propyl gallate, ethoxyquin and the trace element selenium.

The components used according to the invention can be administered by, commercially available, inhaler devices. Compressed NO gas may be obtained from a commercial supplier, typically as a mixture of 200-2000 ppm NO in pure N₂ gas. Said NO-N₂ gas mixture may be delivered into the inhalation gas in an amount of 1 - 100000 nmol/min or, may be mixed with air, oxygen or another suitable carrier gas or gas mixture, generally to a concentration of 1 ppm to 180 ppm of said mixture. For inhalations during extended periods of time a range of 1-40 ppm is generally utilized, while 1-80 ppm or 1-180 ppm may be used for shorter periods of time, when an immediate strong effect is desired. Especially preferable ranges in the last-mentioned cases are 40-80 ppm or 40-180 ppm, respectively. As to further details concerning inhalation of NO reference is made to the prior art, e.g. EP 560 928 B1, in this respect.

The nitric oxide and the superoxide anion scavenger dan be administered sequentially in any order, or they can be administered simultaneously, in the latter case either with the two components from separate sources at the same time or together, or, in the form of a composition comprising both said nitric oxide and said superoxide anion scavenger.

The superoxide anion scavenger can be administered in the same manner as NO, i.e. by inhalation, but also by other common administration routes for pharmaceuticals. Among such routes reference can be made to sublingual, oral, and rectal administrations, application to epithelial surfaces, and injection, which may be subcutaneous, intramuscular, intravenous or intraperitoneal. Preferably, however, the superoxide anion scavenger is administered by inhalation. Thus it may be administered as a powder(i.e. finely divided solid, either provided pure or as a mixture with a biologically compatible carrier powder, or with one or more additional therapeutic compounds) or as a liquid(i.e. dissolved or suspended in a biologically compatible liquid carrier, optionally mixed with one or more additional therapeutic compounds), but can conveniently be inhaled in nebulized form (preferably including particles or droplets having a diameter of less than 10 µm). Carrier liquids and powders that are suitable for inhalation are commonly used in traditional asthma inhalation therapeutics and thus well known in the art.

The superoxide anion scavenger is used in a therapeutically effective amount, such an amount being easily established by the skilled artisan, dependent inter alia on the type of compound used and the route of administration. As should be apparent to a person skilled in the art the term "therapeutic" in this respect as well as in general in the description and claims encompasses prophylactic treatment as well as treatment of an established condition. Furthermore, "therapeutically effective" is utilized in a sense that is common within this technical field, as is e.g. defined in EP 560 928 B1 referred to above, although in general in this specific case the superoxide anion scavenger is therapeutically effective as soon as it reverses a negative effect obtained when using nitric oxide only or enhances the effect of nitric oxide alone. However, as a guidance it can be added that for superoxide dismutase an effective dose range may for instance be 1 000-50 000 units per kg body weight (U/kg), 5 000-15 000 U/kg being a preferable range and 8 000-12 000 U/kg being especially preferred. For other superoxide anion scavengers similar scavenging effects can easily be experimentally established by a person skilled in the art.

With reference to specific and preferable embodiments of said preparation reference is also made to said specific and preferable embodiments of the use according to the invention.

### Brief description of the drawing

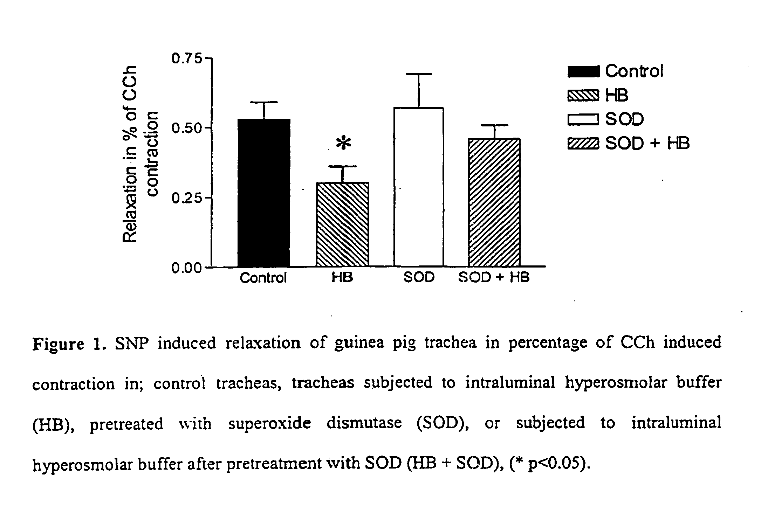

Fig. 1: Relaxation of carbachol-contracted (CCh) trachea by the nitric oxide donor sodium nitroprusside (SNP) after increasing the intraluminal osmolarity to 450 mOsm. Control group treated with hyperosmolarity before and during the experiment and group pretreated with 100 u/ml superoxide dismutase (SOD) on the extraluminal side. Values are per cent relaxation of CCh contracted trachea in mean ± s.e.mean. * significantly different from CCh.

The invention will now be illustrated by the following non-limiting examples:

### Example 1

An in vitro guinea pig tracheal perfusion method was used to investigate the effect of superoxide dismutase (SOD), a scavenger of superoxide anions, on airway relaxation by the nitric oxide donor sodium nitroprusside (SNP) after elevating the osmolarity on the epithelial side with sodium chloride.

### MATERIAL AND METHODS

### Perfused trachea preparation

The experimental protocol was approved by the regional ethics committee on animal experiments. Male Dunkin Hartley guinea pigs (500-800 g) were given an overdose of pentobarbital (0.1 mg kg⁻¹ body weight intraperitoneally). The tracheas were rapidly dissected free of connective tissue, fat and blood vessels removed and mounted on a stainless steel perfusion holder. The perfusion system used was an improved version of Fedan & Frazer's system (Fedan JS, Frazer DG. Influence of epithelium on the reactivity of guinea pig isolated, perfused trachea to bronchoactive drugs. J.Pharmacol.Exp.Ther. 1992; 262:741-750), described earlier by Munakata (Munakata M, Mitzner W, Menkes HA. Osmotic stimuli induce epithelial-dependent relaxation in the guinea pig trachea. J.Appl.Physiol. 1988; 64:466-471). After centrally fixed side-hole catheters were inserted into the lumen of the trachea from each end, the trachea was stretched to its in situ length and placed vertically in a 25 ml extraluminal organ bath containing Krebs-Henseleit buffer. The catheter at the inlet of the trachea, the proximal end, was connected to one side of a differential pressure transducer (P300D, Validyne Engineering Cooperation, CA., USA) and the outlet catheter at the distal end of the trachea was connected to the other side of the transducer. The inside of the trachea was perfused with Krebs- Henseleit buffer from the 25 ml intraluminal bath at 26 ml min⁻¹ in a recirculating loop. The Krebs-Henseleit buffer in both the extra- and intraluminal baths was kept at 37°C and bubbled with a gas mixture of 95% O₂ and 5% CO₂. The transmural pressure was adjusted to correspond to zero at baseline. Responses of the trachea were recorded by a computer (LabView 3.0 software, National Instruments Austin, TX, USA) using a specially designed program (kindly donated by Astra Draco, Lund, Sweden) adapted to the system.
Responses are expressed as the difference in pressure between proximal and distal recording sites (ΔP, cmH₂O).

### Solutions and reagents

The modified isotonic Krebs-Henseleit buffer had an osmolarity of 290 mOsm, and was composed of (in mM): NaCl, 117; NaHCO₃, 25; KH₂PO₄, 1.2; MgSO₄, 1.2; KCl, 4.7; CaCl₂, 2.5 and glucose, 1.03 (pH 7.4, 37°C). The hyperosmolar buffer was formed by increasing the NaCl concentration to 203 mM, which increased the osmolarity to 450 mOsm. Carbachol (Sigma Chemical Co, MO, USA) was dissolved in saline. The nitric oxide donor sodium nitroprusside (SNP, Sigma) and superoxide dismutase (SOD, from bovine liver, Sigma) was dissolved in Krebs-Henseleit buffer. SNP was protected from light until use.

### Experimental protocol

All protocols started with an equilibration period of 60-75 min with washes every 15 min when both intraluminal and extraluminal solutions were replaced for fresh buffer. Thereafter the tracheas were subjected to one of the following protocols:
1) Control (n=6). The intra- and extraluminal buffers were isoosmolar and the trachea was contracted with 1 µM carbachol (CCh) applied extraluminally, corresponding to 50% maximum contraction (as determined in earlier experiments, data not shown). The CCh-contracted trachea was relaxed by the nitric oxide donor SNP, 3 mM added to the intraluminal bath. After a 60 min washout period, in which both the intra- and extraluminal buffer was replaced by fresh buffer every 15 min, the procedure was repeated.
2) Hyperosmolar buffer (n=6). Tracheas were contracted to 50% of maximum contraction by applying 1 µM carbachol extraluminally. The trachea was relaxed by the nitric oxide donor SNP 3 mM added to the intraluminal bath. After a 60 min washout period, the intraluminal buffer was replaced by hyperosmolar buffer 10 min before the second carbachol contraction, then SNP was given to the trachea during hyperosmolar conditions.
3) Superoxide dismutase SOD (n=5). Tracheas were treated as the control group, with one addition to the protocol; SOD (100 units/ml) was applied to the extraluminal side of the trachea 10 min before the second provocation with CCh and SNP.
4) Hyperosmolar buffer and SOD. The enzyme superoxide dismutase (SOD) was used to investigate the effect of superoxide anions on the relaxing effect of SNP. After a first control challenge where 3 mM SNP was used to relax the CCh-contracted trachea, followed by a 60 min washout period, 100 u/ml SOD was applied to the extraluminal side of the trachea (n=8) 10 min before changing the intraluminal buffer to hyperosmolar buffer. The trachea was thereafter contracted with 1 µM CCh and the effect of 3 mM SNP was studied.

### Statistics

Statistical analysis was performed with Statistica software (version 5.0, StatSoft. Inc., Tulsa, OK, USA). The results were analysed with Wilcoxon non-parametric matched pairs test and Mann-Whitney U-test where appropriate. For analysis between groups, Kruskal-Wallis ANOVA (analysis of variance) was used. Results are presented as mean values ± s.e.mean. A statistical result with P<0.05 was considered to be significant.

### RESULTS

The initial CCh induced contraction of the trachea was of the same magnitude in the different groups studied (ns), and all groups relaxed on subsequent SNP (p<0.05) to the same level (ns). This was a control to see that all tracheas were viable and had the same initial prerequisites.
1) Control. CCh produced a contraction of the trachea by 1.49 ± 0.30 cmH2O, subsequent exposure to SNP relaxed the airway by 53.1 ± 6%, i.e. to 0.68 ±0.11 cmH2O (see figure 1).
2) Hyperosmolar buffer. After increasing the osmolarity of the intraluminal buffer to 450 mOSM, the CCh contraction was smaller (0.83 ± 0.17 cmH2O, p<0.05) than in normal conditions. When tracheas were subjected to intraluminal hyperosmolarity SNP relaxed the CCH-contracted trachea by 31 ± 7% (see figure 1). This was a significantly smaller percentual decrease than in the iso-osmolar control conditions (see control group, p<0.05) (see figure 1).
3) SOD. SOD had no effect on the baseline tone and there was no difference between SOD treated and untreated tracheas with respect to CCh contraction (SOD-treated: 1.36 ± 0.15 cmH2O; untreated see above: 1) control) or SNP relaxation (SOD-treated: 57 ±12%; untreated see above: 1) control) (see figure 1).
4) Hyperosmolar buffer and SOD. In tracheas pretreated with SOD before subjecting the intraluminal side to hyperosmolarity, SNP relaxed the trachea by 46 ± 5%, from 1.67 ± 0.29 to 0.92 ± 0.19 cmH2O. This relaxation was not different from the relaxation of SOD pretreated tracheas in isoosmolar conditions, and it was a significantly larger relaxation than in tracheas subjected to hyperosmolar buffer not pretreated with SOD (p<0.05) (see figure 1).

### Example 2

The results in the organ perfusion bath have also been reproduced in in vivo experiments. Thus, rabbits were anesthetized by intramuscular injection of the fluanisone and fentanyl citrate and by intravenous injection of diazepam and paralysed with pancuronium bromide. They were intubated and ventilated with a servo-ventilator (Siemens 900). The inspired oxygen fraction was 0,5. The animals were allocated to four different groups: 1/ Metacholine (MCh) group, exposed to an airway provocation of 1 mg MCh/ml; 2/ NO-MCh group where inhalation of 80 ppm NO was commenced before the MCh provocation and maintained for the remainder of the experiment; 3/ Hypertonic Saline (HS) - NO - MCh group, where hypertonic saline (3,6%) was nebulized into the airways followed by NO inhalation and MCh challenge; and 4/ SOD- HS- NO- MCh group where 8 000 U/kg of SOD was nebulized prior to the nebulization of HS and inhalation of NO, followed by the MCh challenge.

Measurements of respiratory resistance, calculated from airway pressure and gas flow recording, were calculated at baseline and after the MCh challenge in each group.

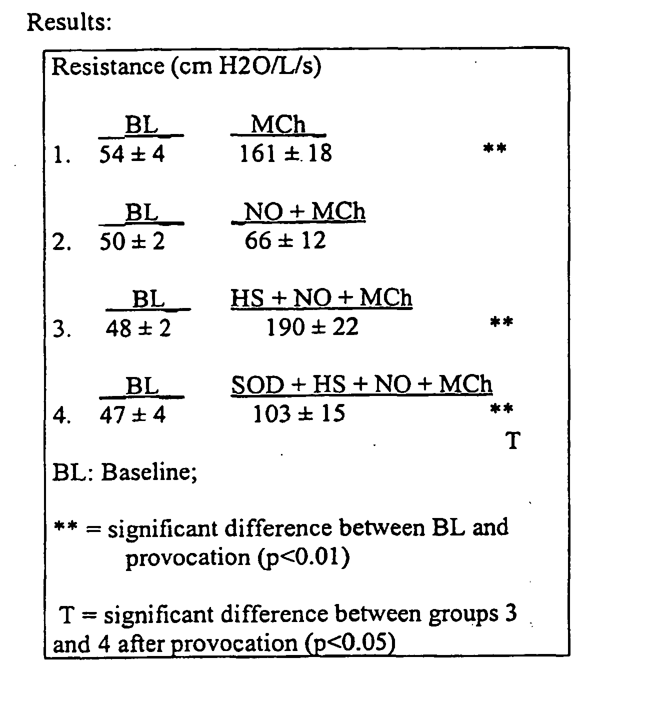

Discussion: It can thus be seen that the rabbits react with a strong airway constriction on MCh challenge with a threefold increase in resistance (group 1). The NO inhalation prevented such increase (group 2)(a slight, non-significant increase was seen). Also, nebulization of HS into the airways abolished completely the effect of NO inhalation (group 3). Thus, the increase in resistance was of the same magnitude as in the first group, exposed solely to MCh. Finally, in the group receiving SOD by nebulization, the increase in resistance was blunted, despite that NO had been given after administration of hypertonic saline (group 4). Thus the increase in resistance was only half that seen the group exposed to hypertonic saline but not SOD.

The problem with non-responder phenomenon to inhaled nitric oxide has been investigated earlier. It was shown that hyperosmolarity on the epithelial side of the airway attenuates the effect of inhaled nitric oxide (Högman M, Hjoberg J, Hedenstierna G. Increased airway osmolarity inhibits the action of nitric oxide in the rabbit. Eur.Respir.J. 1998; 12:1313-1317) and that this is likely to be due to an inactivation of the nitric oxide molecule (Hjoberg J, Högman M, Hedenstierna G. Hyperosmolarity reduces the relaxing potency of nitric oxide donors in guinea-pig trachea. Br.J.Pharmacol. 1999; 127:391-396). According to the present example it was found that preventing the production of superoxide from guinea pig trachea subjected to an increased osmolarity on the epithelial side will increase the relaxation of the pre-contracted trachea by the nitric oxide donor sodium nitroprusside.

## Claims

1. Use of inhalable nitric oxide (NO), in the form of gaseous nitric oxide or a nitric oxide donor, in combination with a superoxide anion scavenger for the manufacture of a medicament for treating airway constriction in a mammal, especially man, said combination being used in a therapeutically effective amount to accomplish relaxation of said airway constriction.

2. Use according to claim 1, wherein said medicament is an inhalable medicament.

3. Use according to any one of claims 1-2, wherein said airway constriction is the result of an airway hyperreactivity or asthma.

4. Use according to claim 3, wherein said airway constriction is associated with asthma bronchiale.

5. Use according to claim 4, wherein said airway, constriction is associated with an acute condition of asthma bronchiale.

6. Use according to claim 4, wherein said airway constriction is associated with status asthmaticus.

7. Use according to any one of claims 1-6, wherein said mammal is a non-responder to inhalation of nitric oxide or nitric oxide donor only.

8. Use according to any one of claims 1-7, wherein said manufacture relates to a medicament for sequential administration of said nitric oxide and said superoxide anion scavenger in any order.

9. Use according to any one of claims 1-7, wherein said medicament is in the form of a composition comprising said nitric oxide and said superoxide anion scavenger for simultaneous administration thereof.

10. Use according to any one of the preceding claims, wherein said superoxide scavenger is selected from the group consisting of enzymes and antioxidants having the ability of removing superoxide anions.

11. Use according to claim 10, wherein the superoxide anion scavenger is superoxide dismutase.

12. Use according to claim 11, wherein the dose of superoxide dismutase is within the range of 1 000-50 000 U/kg, preferably 5 000-15 000 U/kg and most preferably 8 000-12 000 U/kg.

13. Use according to claim 10, wherein said superoxide scavenger is selected from the group consisting of vitamin E, vitamin C, bilirubin, urate, butylated hydroxytoluene, butylated anisole, propyl gallate, ethoxyquin and selenium.

14. Use according to any one of the preceding claims, wherein NO is present in said medicament in an amount of 1-100 000 nmol/min.

15. Use according to any one of the preceding claims, wherein the concentration of NO to be inhaled is within the range of 1-180 ppm, preferably 1-80 ppm, especially 1-40 ppm, said NO being present in a carrier gas or gas mixture.

16. Use according to any one of the preceding claims, wherein said nitric oxide donor is selected from S-nitroso-N-acetylpenicillamine, S-nitrosocysteine, nitroprusside, nitrosoguanidine, glycerol trinitrate, isoamylnitrite, inorganic nitrite, azide and hydroxylamine.

## Patentansprüche

1. Verwendung von inhalierbarem Stickstoff(II)-oxid (NO), in Form von gasförmigem Stickstoff(II)-oxid oder eines Stickstoff(II)-oxid-Donators, in Kombination mit einem Hyperoxid-Anionenfänger zur Herstellung eines Arzneimittels zur Behandlung von Atemwegsverengungen bei Säugern, insbesondere beim Menschen, wobei die Kombination in einer therapeutisch wirksamen Menge verwendet wird, um eine Relaxation der Atemwegsverengung zu erreichen.

2. Verwendung nach Anspruch 1, **dadurch gekennzeichnet, dass** das Arzneimittel ein inhalierbares Arzneimittel ist.

3. Verwendung nach einem der Ansprüche 1 - 2, **dadurch gekennzeichnet, dass** die Atemwegsverengung die Folge von Atemwegshyperreaktivität oder Asthma ist.

4. Verwendung nach Anspruch 3, **dadurch gekennzeichnet, dass** die Atemwegsverengung mit Asthma bronchiale verbunden ist.

5. Verwendung nach Anspruch 4, **dadurch gekennzeichnet, dass** die Atemwegsverengung mit einem akuten Zustand von Asthma bronchiale verbunden ist.

6. Verwendung nach Anspruch 4, **dadurch gekennzeichnet, dass** die Atemwegsverengung mit Status asthmaticus verbunden ist.

7. Verwendung nach einem der Ansprüche 1 - 6, **dadurch gekennzeichnet, dass** der Säuger ein Non-Responder auf die Inhalation von nur Stickstoff(II)-oxid oder Stickstoff(II)-oxid-Donator ist.

8. Verwendung nach einem der Ansprüche 1 - 7, **dadurch gekennzeichnet, dass** die Herstellung ein Arzneimittel zur aufeinanderfolgenden Verabreichung des Stickstoff(II)-oxids und des Hyperoxid-Anionenfängers in beliebiger Reihenfolge betrifft.

9. Verwendung nach einem der Ansprüche 1 - 7, **dadurch gekennzeichnet, dass** das Arzneimittel in Form einer Zusammensetzung, die das Stickstoff(II)-oxid und den Hyperoxid-Anionenfänger aufweist, zu deren gleichzeitiger Verabreichung vorliegt.

10. Verwendung nach einem der vorhergehenden Ansprüche, **dadurch gekennzeichnet, dass** der Hyperoxid-Anionenfänger aus der Gruppe gewählt ist, die aus Enzymen und Antioxidationsmitteln besteht, die Hyperoxid-Anionen eliminieren können.

11. Verwendung nach Anspruch 10, **dadurch gekennzeichnet, dass** der Hyperoxid-Anionenfänger Hyperoxid-Dismutase ist.

12. Verwendung nach Anspruch 11, **dadurch gekennzeichnet, dass** die Dosis von Hyperoxid-Dismutase in dem Bereich von 1.000 - 50.000 E/kg, vorzugsweise 5.000 - 15.000 E/kg und am besten 8.000 - 12.000 Elkg, liegt.

13. Verwendung nach Anspruch 10, **dadurch gekennzeichnet, dass** der Hyperoxid-Anionenfänger aus der Gruppe gewählt ist, die aus Vitamin E, Vitamin C, Bilirubin, Urat, butyliertes Hydroxytoulen, butyliertes Anisol, Propylgallat, Ethoxyquin und Selen besteht.

14. Verwendung nach einem der vorhergehenden Ansprüche, **dadurch gekennzeichnet, dass** NO in dem Arzneimittel in einer Menge von 1 - 100.000 nmol/min vorliegt.

15. Verwendung nach einem der vorhergehenden Ansprüche, **dadurch gekennzeichnet, dass** die Konzentration des zu inhalierenden NO in dem Bereich von 1 - 180 ppm, vorzugsweise 1 - 80 ppm, insbesondere 1 - 40 ppm, liegt, wobei das NO in einem Trägergas oder Gasgemisch vorliegt.

16. Verwendung nach einem der vorhergehenden Ansprüche, **dadurch gekennzeichnet, dass** der Stickstoff(II)-oxid-Donator aus S-Nitroso-N-acetylpenicillamin, S-Nitrosocystein, Nitroprussid, Nitrosoguanidin, Glyceroltrinitrat, Isoamylnitrit, anorganischem Nitrit, Azid und Hydroxylamin gewählt ist.

## Revendications

1. Utilisation d'oxyde nitrique susceptible d'être inhalé (NO), sous forme d'oxyde nitrique gazeux ou d'un donneur d'oxyde nitrique, en combinaison avec un piège à anions super-oxyde pour la fabrication d'un médicament pour traiter une constriction des voies aériennes chez un mammifère, en particulier l'homme, ladite combinaison étant utilisée dans une quantité efficace au plan thérapeutique pour réaliser un relâchement de ladite constriction des voies aériennes.

2. Utilisation selon la revendication 1, dans laquelle ledit médicament est un médicament susceptible d'être inhalé.

3. Utilisation selon l'une quelconque des revendications 1 à 2, dans laquelle ladite constriction des voies aériennes est le résultat d'une hyperréactivité des voies aériennes ou de l'asthme.

4. Utilisation selon la revendication 3, dans laquelle ladite constriction des voies aériennes est associée à l'asthme bronchique.

5. Utilisation selon la revendication 4, dans laquelle ladite constriction des voies aériennes est associée à un état aigu de l'asthme bronchique.

6. Utilisation selon la revendication 4, dans laquelle ladite constriction des voies aériennes est associée à un état de mal asthmatique.

7. Utilisation selon l'une quelconque des revendications 1 à 6, dans laquelle ledit mammifère ne présente pas de réponse à l'inhalation uniquement d'oxyde nitrique ou d'un donneur d'oxyde nitrique.

8. Utilisation selon l'une quelconque des revendications 1 à 7, dans laquelle ladite fabrication concerne un médicament pour une administration successive dudit oxyde nitrique et dudit piège à anions super-oxyde dans un ordre quelconque.

9. Utilisation selon l'une quelconque des revendications 1 à 7, dans laquelle ledit médicament est sous forme d'un composition comprenant ledit oxyde nitrique et ledit piège à anions super-oxyde pour une administration simultanée de ceux-ci.

10. Utilisation selon l'une quelconque des revendications précédentes, dans laquelle ledit piège à super-oxyde est choisi dans le groupe constitué par les enzymes et les antioxydants présentant la capacité d'élimination des anions super-oxyde.

11. Utilisation selon la revendication 10, dans laquelle le piège à anions super-oxyde est la superoxyde dismutase.

12. Utilisation selon la revendication 11, dans laquelle la dose de superoxyde dismutase est dans la gamme de 1 000 à 50 000 U/kg, de préférence de 5 000 à 15 000 U/kg et tout particulièrement de 8 000 à 12 000 U/kg.

13. Utilisation selon la revendication 10, dans laquelle ledit piège à anions super-oxyde est choisi dans le groupe constitué par la vitamine E, la vitamine C, la bilirubine, l'urate, l'hydroxytoluène butylé, l'anisole butylé, le gallate de propyle, l'éthoxyquine et le sélénium.

14. Utilisation selon l'une quelconque des revendications précédentes, dans laquelle le NO est présent dans ledit médicament dans une quantité de 1 à 100 000 nmol/min.

15. Utilisation selon l'une quelconque des revendications précédentes, dans laquelle la concentration du NO à inhaler est dans la gamme de 1 à 180 ppm, de préférence de 1 à 80 ppm, en particulier de 1 à 40 ppm, ledit NO étant présent dans un véhicule gazeux ou dans un mélange de gaz.

16. Utilisation selon l'une quelconque des revendications précédentes, dans laquelle ledit donneur d'oxyde nitrique est choisi parmi la S-nitroso-N-acétylpénicillamine, la S-nitrosocystéine, la nitroprusside, la nitrosoguanidine, la trinitrate de glycérol, l'isoamylnitrite, le nitrite minéral, l'azoture et l'hydroxylamine.
